(19) Europäisches Patentamt
European Patent Office
Office européen des brevets

(11) Veröffentlichungsnummer: **0 145 661**
**A2**

(12) # EUROPÄISCHE PATENTANMELDUNG

(21) Anmeldenummer: **84810578.9**

(51) Int. Cl.[4]: **C 07 D 213/64**

(22) Anmeldetag: **28.11.84**

(30) Priorität: **02.12.83 CH 6468/83**
**09.11.84 CH 5388/84**

(43) Veröffentlichungstag der Anmeldung: **19.06.85**
**Patentblatt 85/25**

(84) Benannte Vertragsstaaten: **BE CH DE FR GB IT LI NL**

(71) Anmelder: **CIBA-GEIGY AG, Postfach, CH-4002 Basel (CH)**

(72) Erfinder: **Francotte, Eric, Dr., Violaweg 73/10, CH-4303 Kaiseraugst (CH)**
Erfinder: **Ackermann, Peter, Dr., Hangelimattweg 113, CH-4148 Pfeffingen (CH)**

(54) **Neue Alkohole.**

(57) Enantiomere von 1-(6-Phenoxy-2-pyridyl)-äthanolen der Formel:

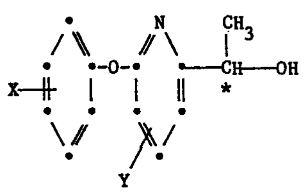

(I)

worin * (+) oder (−) und X und Y unabhängig voneinander Wasserstoff, Halogen, Nitro, $C_1$-$C_4$-Alkyl, $C_1$-$C_4$-Halogenalkyl, $C_1$-$C_4$-Alkoxy, $C_2$-$C_5$-Alkenyl oder $C_2$-$C_5$-Alkinyl bedeuten.

Es werden Verfahren zur Herstellung dieser Enantiomeren und ihre Verwendung zur Herstellung von bioziden Verbindungen beschrieben.

EP 0 145 661 A2

CIBA-GEIGY AG
Basel (Schweiz)

5-14679/1+2/=

Neue Alkohole

Die vorliegende Anmeldung betrifft neue Enantiomere von 1-(6-Phenoxy-2-pyridyl)-äthanolen, Verfahren zu ihrer Herstellung und ihre Verwendung zur Synthese von bioziden Verbindungen. Diese Enantiomeren haben die Formel

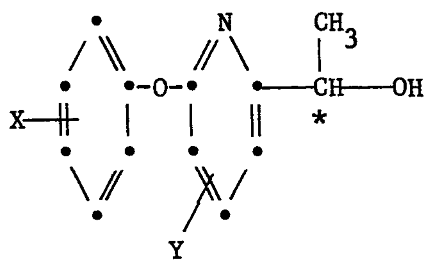

(I)

worin * (+) oder (-) und X und Y unabhängig voneinander Wasserstoff, Halogen, Nitro, $C_1$-$C_4$-Alkyl, $C_1$-$C_4$-Halogenalkyl, $C_1$-$C_4$-Alkoxy, $C_2$-$C_5$-Alkenyl oder $C_2$-$C_5$-Alkinyl bedeuten.

Unter Halogen ist dabei Fluor, Chlor, Brom oder Jod zu verstehen.

Die bei X und Y in Frage kommenden Alkyl-, Halogenalkyl-, Alkoxy-, Alkenyl- oder Alkinylgruppen können geradkettig oder verzweigt sein. Beispiele solcher Gruppen sind: Methyl, Methoxy, Trifluormethyl, Aethyl, Aethoxy, Propyl, Isopropyl, n-Butyl, Vinyl, 1-Propenyl, Aethinyl und 1-Propinyl.

Bevorzugt sind Enantiomere der Formel I, worin * (+) oder (-), X Wasserstoff, Halogen, Nitro oder $-C{\equiv}CH$ und Y Wasserstoff bedeuten.

Insbesondere bevorzugt sind Enantiomere der Formel I worin * (+) oder (-), X Wasserstoff oder Halogen und Y Wasserstoff bedeuten.

Es ist an sich bekannt, dass man die Entantiomeren der Formel I durch Auftrennung von racemischen 1-(6-Phenoxy-2-pyridyl)-äthanolen an optisch aktiven Trägermaterialien erhalten kann. Die richtige Wahl des Trägermaterials ist dabei ausschlaggebend für eine erfolgreiche Trennung. Ueberraschenderweise wurde nun gefunden, dass sich racemische 1-(6-Phenoxy-2-pyridyl)-äthanole oder deren Acetate chromatographisch an mikrokristallinen Cellulosen insbesondere an mikrokristallinen Triacetylcellulosen sowie Tribenzoylcellulosen in die Enantiomeren der Formel I auftrennen lassen.

Racemische 1-(6-Phenoxy-2-pyridyl)-äthanole sind bekannt (U.S.P. 4,323,574) oder können nach bekannten Methoden hergestellt werden. Aus den optisch aktiven Alkoholen der Formel I lassen sich aber überraschenderweise durch Umsetzung mit für Pyrethroide üblichen Säuren Ester von stärkerer insektizider und akarizider Wirkung herstellen, als mit den bekannten racemischen 1-(6-Phenoxy-2-pyridyl)-äthanolen. Zur Umsetzung mit den Enantiomeren 1-(6-Phenoxy-2-pyridyl)-äthanolen der Formel I können beispielsweise folgende Säuren benützt werden:

a) 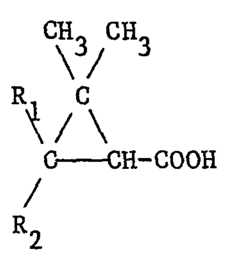

worin $R_1$ Wasserstoff,

$R_2$ 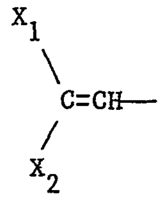 oder 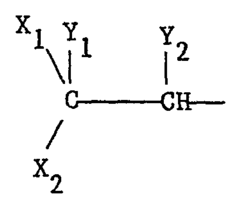

worin,

$X_1$ Methyl oder Halogen,

$X_2$ Methyl, Trifluormethyl, Halogen oder p-Chlorphenyl,

$Y_1$ Halogen und

$Y_2$ Wasserstoff oder Halogen bedeuten.

b) 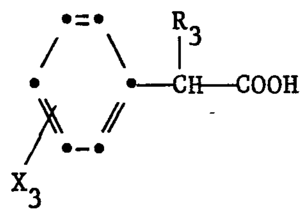

worin,

$X_3$ Wasserstoff, Halogen, $C_1$-$C_4$-Alkyl, $C_1$-$C_4$-Alkoxy, $C_1$-$C_4$-Halogenalkyl, $C_1$-$C_4$-Halogenalkoxy, $-COCH_3$ oder Methylendioxy und

$R_3$ Isopropyl oder Cyclopropyl bedeuten.

<u>Beispiel 1:</u> Herstellung der Enantiomeren durch chromatographische Spaltung des racemischen Alkohols der Formel

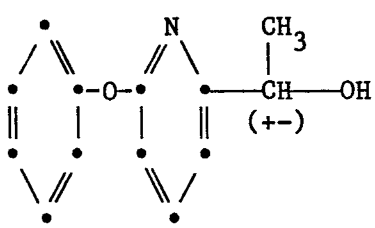

Mit 36 g Triacetylcellulose [Herstellung nach bekannten Verfahren: Chromatographia <u>6</u>, 277 (1973)] und Aethanol/Wasser 95/5 wird eine Glassäule (1,25 cm x 58,5 cm) gefüllt. An diesem Träger werden 6,5 mg Racemat des Alkohols der Formel

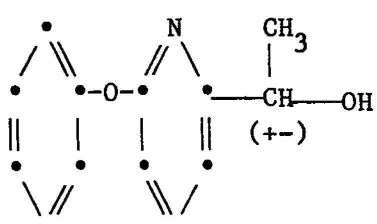

aufgetragen und chromatographiert.

Das Racemat wird mit Aethanol/Wasser 95/5 bei einem Druck von 13 Bar chromatographiert, wobei die Fliessgeschwindigkeit von 24 ml/Std. resultiert. Das Eluat wird über Durchflussküvetten eines Polarimeters (Perkin Elmer, Typ 241 MC) und eines UV-Spektrophotometers (Shimadzu UV-120-02) geleitet. Ein Zweikanalschreiber registriert Substanzkonzentration und Drehwert.

Die Drehwerte der beiden Enantiomeren werden aus der Elutionskurve berechnet.

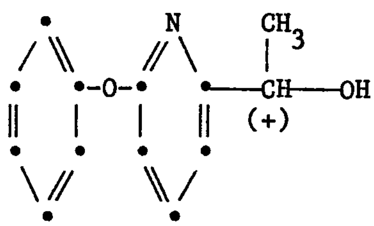

$[\alpha]_{365} = + 89° \pm 2°$
(c = 0,033; Aethanol/$H_2O$ 95:5)

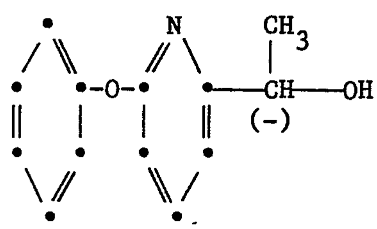

$[\alpha]_{365} = - 91° \pm 2°$
(c = 0,042; Aethanol/$H_2O$ 95:5)

Trennfaktor $\alpha = \frac{V_2 - V_0}{V_1 - V_0} = 1,25.$

<u>Beispiel 2:</u> Herstellung der Enantiomeren durch chromatographische Spaltung des racemischen Alkohols der Formel

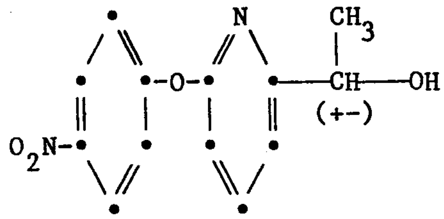

Das Racemat wird an einer Tribenzoylcellulose-Säule (0,4 cm x 25 cm) mit einem Gemisch von Hexan/Isopropyl (9:1) bei einer Fliessgeschwindigkeit von 0,2 ml/Min. chromatographiert.

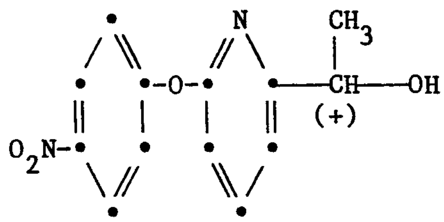

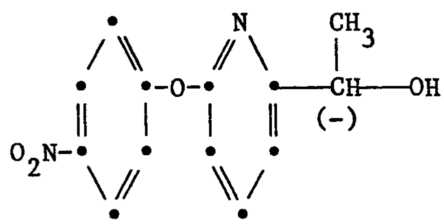

Trennfaktor = 1,15

$R_s$ = Auflösungsfaktor = 1,0

<u>Beispiel 3:</u> Herstellung der Enantiomeren des racemischen Alkohols der Formel

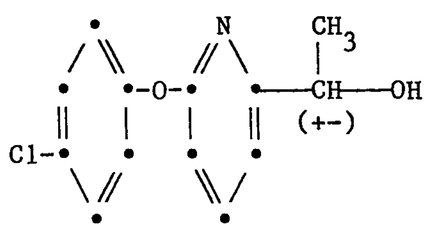

a) Chromatographische Racemattrennung des Acetats.

In einer Triacetylcellulose Säule (1,25 x 30 cm; 14 g Triacetylcellulose) werden 20 mg des racemischen Acetats mit Aethanol/Wasser (95/5) als Eluiermittel bei einer Fliessgeschwindigkeit von 33 ml/Std. chromatographiert. Die Drehwerte der beiden enantiomeren Acetate werden gemessen.

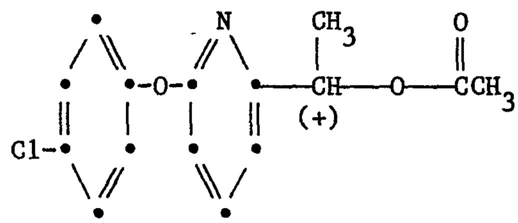

$[\alpha]_{436} = + 77° \pm 3°$
(c = 0,38; Aethanol)

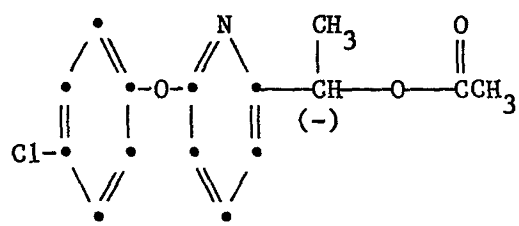

$[\alpha]_{436} = - 76° \pm 3°$
(c = 0,38; Aethanol)

Trennfaktor = 7,9

b) Verseifung der Acetate

5 mg des optisch reinen (+) bzw. (-) Acetats (Bsp. 3a) werden in 0,3 ml eines Gemisches von Aethanol/Wasser (10/7) mit 15 mg $K_2CO_3$ 1 Stunde am Rückfluss gekocht. Das Gemisch wird mit 3 ml Chloroform verdünnt und über Natriumsulfat getrocknet. Nach dem

Filtrieren und Eindampfen der Lösung wird der Rückstand über Kieselgel chromatographisch gereinigt (Laufmittel: Chloroform). Man erhält die Enantiomeren der Formeln:

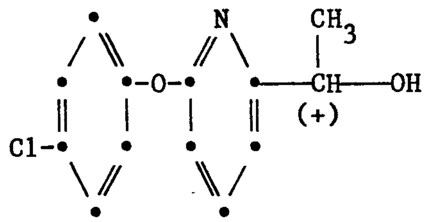

$[\alpha]_{436}$ = + 48°± 3°
(c = 0,23; Aethanol)

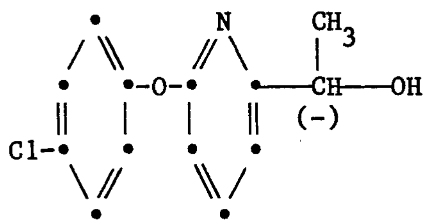

$[\alpha]_{436}$ = - 45°± 3°
(c = 0,21; Aethanol)

Auf analoge Weise erhält man auch folgende enantiomere Acetate und Alkohole:

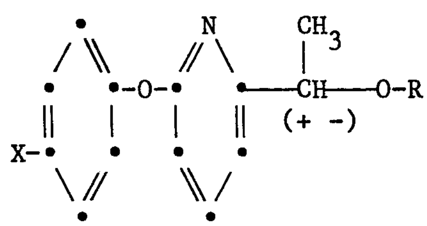

| X | R=$\overset{\overset{O}{\Vert}}{C}CH_3$ | | R=H |
|---|---|---|---|
| | Trennfaktor | $[\alpha]_{436}$ (Aethanol) | $[\alpha]_{436}$ (Aethanol) |
| F | 1,36 | + 96° ± 3° (C = 0,41)<br>- 99° ± 3° (C = 0,53) | + 54° ± 3° (C = 0,30)<br>- 51° ± 3° (C = 0,39) |
| Br | 1,80 | + 72° ± 3° (C = 0,85)<br>- 71° ± 3° (C = 0,96) | + 48° ± 3° (C = 0,74)<br>- 49° ± 3° (C = 0,79) |
| -C≡CH | 1,87 | + 91° ± 3° (C = 0,65)<br>- 92° ± 3° (C = 0,97) | + 72° ± 3° (C = 0,71)<br>- 77° ± 3° (C = 0,54) |

Beispiel 4: Herstellung des 1-R-cis-3-(2,2-Dichlorvinyl)-2,2-dimethylcyclopropankarbonsäure-R-α-methyl-(6-phenoxy-2-picolyl)--esters.

Zu einer eisgekühlten Lösung von 3,2 g 1-R-cis-3-(2,2-Dichlorvinyl)-2,2-dimethylcyclopropankarbonsäurechlorid in 20 ml Toluol gibt man nacheinander 1,4 g Pyridin, gelöst in 5 ml Toluol und dann 3 g der Verbindung der Formel

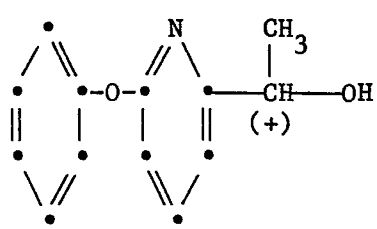

in 10 ml Toluol. Die leicht gelbliche Suspension versetzt man mit 200 mg 4-Dimethylaminopyridin und rührt das Reaktionsgemisch 16 Stunden bei Raumtemperatur. Nach Zugabe von 100 ml Toluol wird die organische Phase mit eisgekühlter 1n HCl-, 10%iger $K_2CO_3$-, gesättigter $NaHCO_3$- und gesättigter Kochsalzlösung gewaschen und

über $MgSO_4$ getrocknet. Das Lösungsmittel wird nun unter vermindertem Druck entfernt und das Rohprodukt an Kieselgel mit Toluol als Eluiermittel gereinigt.

Man erhält den 1-R-cis-3-(2,2-Dichlorvinyl)-2,2-dimethylcyclopropankarbonsäure-R-α-methyl-(6-phenoxy-2-picolyl)-ester (Nr. 1) mit folgenden physikalischen Daten:

$n_D^{20°}$ = 1,5605
$[\alpha]_D$ = + 116° ± 1° [c = 1,07 in Benzol]

Auf analoge Weise werden auch folgende Ester hergestellt:

Nr. 2 1-R-cis-3-(2,2-Dichlorvinyl)-2,2-dimethylcyclopropankarbonsäure-S-α-methyl-(6-phenoxy-2-picolyl)-ester,

$n_D^{20°}$ = 1,5591
$[\alpha]_D$ = - 69° ± 1° [c = 0,937 in Benzol]

Nr. 3 1-R-trans-3-(2,2-Dichlorvinyl)-2,2-dimethylcyclopropankarbonsäure-R-α-methyl(6-phenoxy-2-picolyl)-ester.

$n_D^{20°}$ = 1,5581
$[\alpha]_D$ = + 77° ± 1° [c = 0,74 in Benzol]

Nr. 4 1-R-trans-3-(2,2-Dichlorvinyl)-2,2-dimethylcyclopropankarbonsäure-S-α-methyl(6-phenoxy-2-picolyl)-ester.

$n_D^{21°}$ = 1,5581
$[\alpha]_D$ = - 56° ± 1° [c = 0,83 in Benzol]

Beispiel 5: Insektizide Frassgift-Wirkung:

Baumwollpflanzen werden mit einer Versuchslösung, enthaltend 1,25; 0,6 oder 0,3 g Wirkstoff gemäss Beispiel 4 pro 100 l $H_2O$, besprüht.

Nach dem Antrocknen des Belages werden die Pflanzen mit Larven der Spezies Heliothis virescens ($L_1$-Stadium) besetzt. Man verwendet pro Versuchsverbindung und pro Test-Spezies zwei Pflanzen, und eine Auswertung der erzielten Abtötungsrate erfolgt nach 24 und 48 Stunden. Der Versuch wird bei 24°C und 60% relativer Luftfeuchtigkeit durchgeführt.

Die Verbindung Nr. 1 gemäss dem Herstellungsbeispiel 4 zeigt die in der folgenden Tabelle angegebene Wirkung gegen Larven der Spezies Heliothis virescens:

Mortalität in % der Heliothis virescens $L_1$-Larven

| | 1,25 g | | 0,6 g | | 0,3 g Konz. | |
|---|---|---|---|---|---|---|
| | 24 | 48 | 24 | 48 | 24 | 48 Std. |
| Verbindung Nr 1 gemäss Beispiel 4 | 43 | 90 | 43 | 77 | 28 | 55 |
| Isomerengemisch der Formel * bekannt aus U.S.P. 4,323,574 | 15 | 20 | 0 | 0 | 0 | 0 |

* 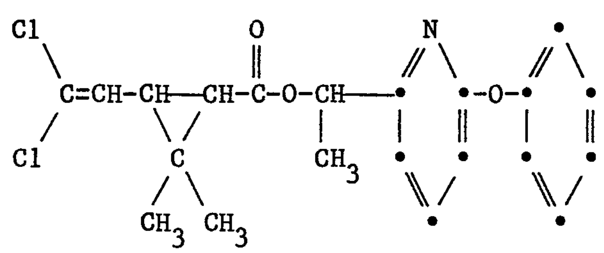 (cis/trans = 9:1)

Patentansprüche

1. Ein Enantiomeres der Formel

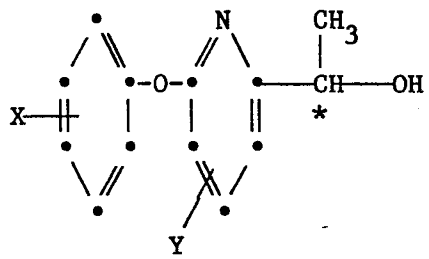

(I)

worin * (+) oder (-) und X und Y unabhängig voneinander Wasserstoff, Halogen, Nitro, $C_1$-$C_4$-Alkyl, $C_1$-$C_4$-Halogenalkyl, $C_1$-$C_4$-Alkoxy, $C_2$-$C_5$-Alkenyl oder $C_2$-$C_5$-Alkinyl bedeuten.

2. Ein Enantiomeres gemäss Anspruch 1, worin X Wasserstoff, Halogen, Nitro oder $-C{\equiv}CH$ und Y Wasserstoff bedeuten.

3. Ein Enantiomeres gemäss Anspruch 2, worin X Wasserstoff oder Halogen und Y Wasserstoff bedeuten.

4. Das Enantiomere gemäss Anspruch 3 der Formel

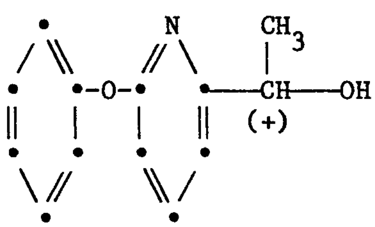

.

5. Das Enantiomere gemäss Anspruch 3 der Formel

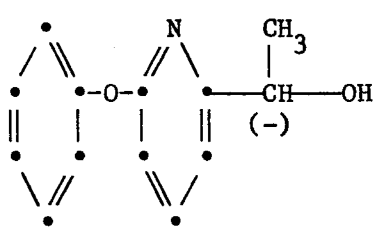

.

6. Das Enantiomere gemäss Anspruch 3 der Formel

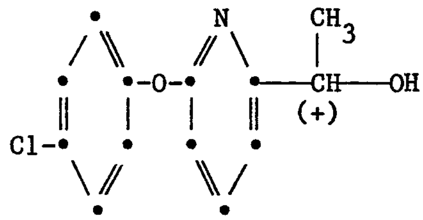

.

7. Das Enantiomere gemäss Anspruch 3 der Formel

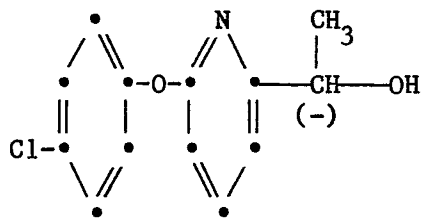

.

8. Das Enantiomere gemäss Anspruch 3 der Formel

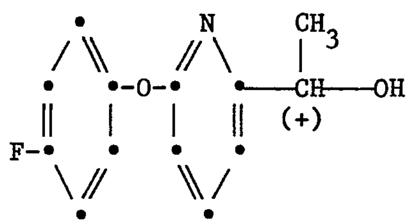

.

9. Das Enantiomere gemäss Anspruch 3 der Formel

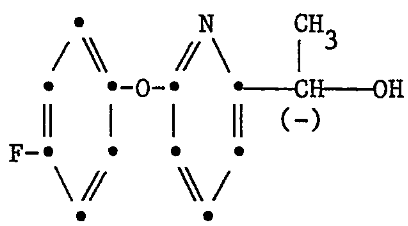

.

10. Das Enantiomere gemäss Anspruch 3 der Formel

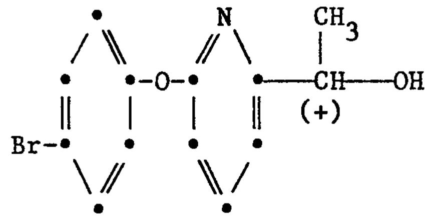

.

11. Das Enantiomere gemäss Anspruch 3 der Formel

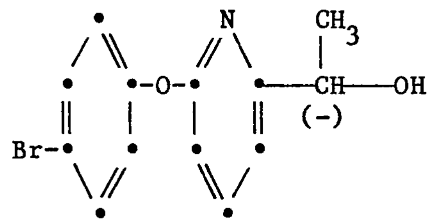

.

12. Das Enantiomere gemäss Anspruch 2 der Formel

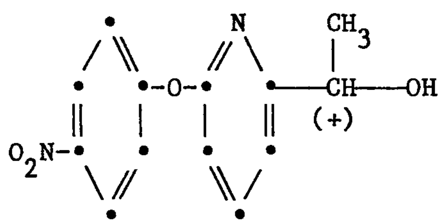

.

13. Das Enantiomere gemäss Anspruch 2 der Formel

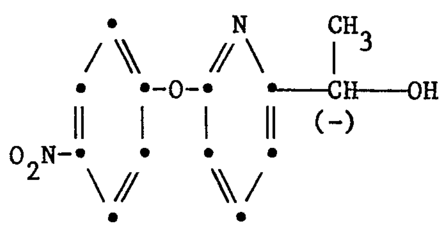

.

14. Das Enantiomere gemäss Anspruch 2 der Formel

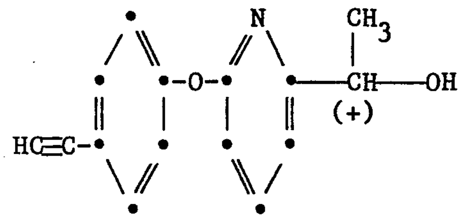

.

15. Das Enantiomere gemäss Anspruch 2 der Formel

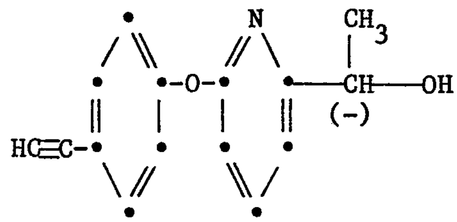

.

16. Verfahren zur Herstellung der Enantiomeren gemäss Anspruch 1, dadurch gekennzeichnet, dass man a) einen racemischen Alkohol der Formel

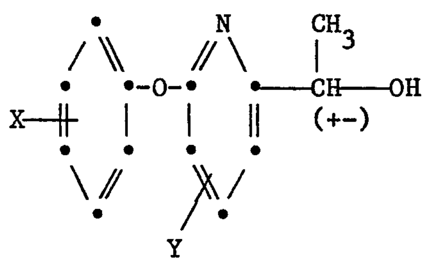

(I)

worin X und Y die im Anspruch 1 angegebene Bedeutung haben, an einer mikrokristallinen Cellulose, insbesondere an einer mikrokristallinen Triacetylcellulose oder Tribenzoylcellulose chromatographisch trennt.

oder b) ein racemisches Acetat der Formel

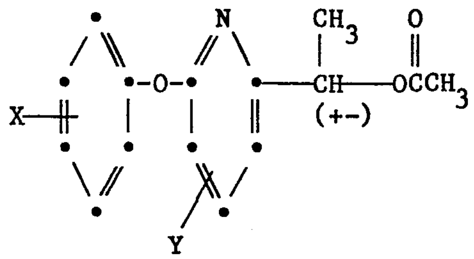

(I)

worin X und Y die im Anspruch 1 angegebene Bedeutung haben, an einer mikrokristallinen Cellulose chromatographisch trennt und anschliessend die erhaltenen reinen enantiomeren Acetate zu den reinen enantiomeren Alkoholen gemäss Anspruch 1 verseift.

17. Die Verwendung eines Enantiomeren gemäss Anspruch 1 zur Herstellung von bioziden Verbindungen.

18. Die Verwendung gemäss Anspruch 17 zur Herstellung von pyrethroiden Verbindungen.

19. Die Verwendung gemäss Anspruch 18 zur Herstellung des 1-R-cis-3-(2,2-Dichlorvinyl)-2,2-dimethylcyclopropankarbonsäure-R-α-methyl-(6-phenoxy-2-picolyl)-, 1-R-cis-3-(2,2-Dichlorvinyl)-2,2-dimethylcyclopropankarbonsäure-S-α-methyl-(6-phenoxy-2-picolyl)-, 1-R-trans-3-(2,2-Dichlorvinyl)-2,2-dimethylcyclopropankarbonsäure-R-α-methyl-(6-phenoxy-2-picolyl)- oder 1-R-trans-3-(2,2-Dichlorvinyl)-2,2-dimethylcyclopropankarbonsäure-S-α-methyl-(6-phenoxy-2-picolyl)-esters.

FO 7.5/WH/we*